# EUROPÄISCHE PATENTSCHRIFT

(11) **EP 2 340 306 B1**
(45) Veröffentlichungstag und Bekanntmachung des Hinweises auf die Patenterteilung: **14.12.2016**
(21) Anmeldenummer: 09740088.1
(22) Anmeldetag: 09.10.2009
(51) Int. Cl.: C12N 15/09

(54) **EXPRESSIONSVERSTÄRKTE NUKLEINSÄUREN**
NUCLEIC ACIDS WITH ENHANCED EXPRESSION CHARACTERISTICS
ACIDES NUCLÉIQUES À EXPRESSION AMPLIFIÉE

(30) Priorität: 21.10.2008 DE 102008052529
(43) Veröffentlichungstag der Anmeldung: 06.07.2011
(73) Patentinhaber: BASF SE, 67056 Ludwigshafen am Rhein (DE)
(72) Erfinder: RADNAI, Victoria, 47053 Duisburg (DE); EVERS, Stefan, 40822 Mettmann (DE); BONGAERTS, Johannes, 41541 Dormagen (DE); MAURER, Karl-Heinz, 40699 Erkrath (DE)
(74) Vertreter: BASF IP Association
(86) Internationale Anmeldenummer: PCT/EP2009/063200
(87) Internationale Veröffentlichungsnummer: WO 2010/046250

(56) Entgegenhaltungen:
- WO-A1-03/095658
- WO-A2-99/43835
- WO-A2-2004/005221
- WO-A2-2008/066931
- WO-A2-2008/140615
- KUNST F ET AL: "The complete genome sequence of the Gram-positive bacterium Bacillus subtilis" NATURE (LONDON), Bd. 390, Nr. 6657, 20. November 1997 (1997-11-20), Seiten 249-256, XP002562068 ISSN: 0028-0836
- REY MICHAEL W ET AL: "Complete genome sequence of the industrial bacterium Bacillus licheniformis and comparisons with closely related Bacillus species." GENOME BIOLOGY, Bd. 5, Nr. 10, 2004, Seiten R77-1-R77-12, XP002562069 ISSN: 1465-6914
- TANAKA T ET AL: "prtR enhances the mRNA level of the Bacillus subtilis extracellular proteases." JOURNAL OF BACTERIOLOGY JUL 1987, Bd. 169, Nr. 7, Juli 1987 (1987-07), Seiten 3044-3050, XP002562070 ISSN: 0021-9193
- ASEEV L V ET AL: "A new regulatory circuit in ribosomal protein operons: S2-mediated control of the rpsB-tsf expression in vivo" RNA 200809 US, Bd. 14, Nr. 9, September 2008 (2008-09), Seiten 1882-1894, XP002563834 ISSN: 1355-8382 1469-9001

## Beschreibung

Die vorliegende Erfindung betrifft Nukleinsäuren mit einem speziellen Tandempromotor, die eine verstärkte Expression einer für ein Polypeptid codierenden Nukleinsäuresequenz in einer Wirtszelle bewirken, sowie Vektoren und Wirtszellen, die eine solche Nukleinsäure enthalten. Ferner betrifft die Anmeldung Verfahren und Verwendungen solcher Nukleinsäuren, Vektoren und Wirtszellen, insbesondere fermentative Verwendungen und Verfahren.

Die vorliegende Erfindung liegt auf dem Gebiet der Biotechnologie, insbesondere der Herstellung von Polypeptiden durch Fermentation von Mikroorganismen, die zur Bildung der interessierenden Wertstoffe, insbesondere Polypeptide, in der Lage sind. Hierfür werden Mikroorganismen eingesetzt, die die Gene der interessierenden Polypeptide exprimieren, so dass es sich zumeist also um transgene, gentechnisch veränderte Organismen (GVO). Diese Mikroorganismen werden auch als Wirtszellen bezeichnet. Die Gene der interessierenden Polypeptide wurden in der Regel in die Wirtszellen derart eingebracht, dass sie auch von diesen exprimiert werden. Häufig liegen sie auf sogenannten Expressionsvektoren zusammen mit Promotorsequenzen (Promotoren) vor, die die Genexpression ermöglichen.

Zur Fermentation von Mikroorganismen besteht ein reichhaltiger Stand der Technik, insbesondere auch im großtechnischen Maßstab; er reicht von der Optimierung der betreffenden Stämme hinsichtlich der Bildungsrate und der Nährstoffausnutzung über die technische Gestaltung der Fermenter bis hin zur Gewinnung der Wertstoffe aus den betreffenden Zellen selbst und/oder dem Fermentationsmedium. Hierfür kommen sowohl genetische und mikrobiologische als auch verfahrenstechnische und biochemische Ansätze zu tragen.

Häufig eingesetzt werden beispielsweise Mikroorganismen der Gattungen Bacillus, Streptomyces, Humicola, Escherichia oder Pseudomonas oder filamentöse Fungi.

Hierbei ist es wünschenswert, eine möglichst hohe Produktausbeute in der Fermentation zu erhalten. Die Produktausbeute ist dabei abhängig von mehreren Faktoren. Beispielsweise bilden die Mikroorganismen neben dem eigentlich gewünschten Produkt üblicherweise eine Vielzahl weiterer Substanzen, an denen in der Regel kein Interesse besteht. Daher gehen diese Syntheseleistungen auf Kosten der Bildung des gewünschten Produktes. Bei der Expression eines Transgens, d. h. eines Gens, das in die Wirtszelle eingebracht wurde, hängt diese und damit die Produktausbeute wesentlich von dem verwendeten Expressionssystem ab. Eine besondere Rolle spielen dabei die Promotoren, die für die Expression des Transgens in der Wirtszelle verwendet werden. Promotoren sind mannigfaltig im Stand der Technik bekannt, jedoch führen nur ausgewählte Promotoren in Kombination mit einem konkreten Transgen in einer Wirtszelle zu einer zufrieden stellenden Expression des Transgens und damit zu einer zufrieden stellenden Produktausbeute.

Zur Steigerung der Produktausbeute wurde in der europäischen Offenlegungsschrift EP 1056873 weiterhin vorgeschlagen, Tandem-Promotoren zur Expression des Gens zu nutzen, die aus zwei ausgewählten Promotoren bestehen. Im Stand der Technik sind zwar eine Vielzahl von Promotoren bekannt, die prinzipiell kombiniert werden könnten für die Expression eines Transgens. Jedoch führt längst nicht jede prinzipiell mögliche Promotorkombination auch zu einer verbesserten im Sinne von gesteigerten Expression eines Transgens, so dass damit eine Steigerung der Produktausbeute erreicht wird.

Aufgabe der Erfindung ist es daher, die Produktausbeute in einer Fermentation zu erhöhen. Insbesondere sollte eine Nukleinsäure zur Verfügung gestellt werden, unter Einsatz derer sich die Expression eines Transgens in einer Wirtszelle und damit die Produktausbeute in einer Fermentation erhöht.

Weitere Aufgaben bestanden in der Bereitstellung von Vektoren, Wirtszellen, Herstellungsverfahren und Verwendungen, unter Einsatz bzw. Anwendung derer sich die Expression eines Transgens und damit die Produktausbeute in einer Fermentation erhöht.

Ein Gegenstand der Erfindung ist somit eine nicht natürliche Nukleinsäure umfassend
a) eine erste Promotorsequenz umfassend eine Nukleinsäuresequenz, die zu SEQ ID NO. 7 zu mindestens 75% identisch ist,
b) eine zweite Promotorsequenz umfassend eine Nukleinsäuresequenz, die zu SEQ ID NO. 6 in den Positionen 7 bis 234 zu mindestens 78,5% identisch ist,
c) eine Nukleinsäuresequenz, die für ein Polypeptid codiert,
dadurch gekennzeichnet, dass sich auf dem Nukleinsäuremolekül in 5'→3'-Orientierung die zweite Promotorsequenz vor der ersten Promotorsequenz befindet.

Eine erfindungsgemäße Nukleinsäure bewirkt daher die Expression der Nukleinsäuresequenz (c) in einem Wirtsorganismus auf Grund der Anwesenheit der ersten und zweiten Promotorsequenzen (a) und (b). Die Expression eines Gens ist dessen Übersetzung in das bzw. die von diesem Gen codierte(n) Genprodukt(e), also in ein Polypeptid (Protein) bzw. in mehrere Polypeptide (Proteine). In der Regel umfasst die Genexpression die Transkription, also die Synthese einer Ribonukleinsäure (mRNA) anhand der DNA(Desoxyribonukleinsäure)-Sequenz des Gens und deren Translation in die entsprechende Polypeptidkette. Die Expression eines Gens führt zur Bildung des entsprechenden Genproduktes, welches häufig eine physiologische Aktivität aufweist und/oder bewirkt und/oder einen Beitrag zu einer übergeordneten physiologischen Aktivität leistet, an der mehrere verschiedene Genprodukte beteiligt sind. Beispiele hierfür sind Enzyme. Das Genprodukt kann jedoch auch eine strukturgebende Wirkung haben. Beispiele hierfür sind Proteine der Zellmembran oder der Zellwand oder des Zytoskeletts. Die Begriffe Polypeptid und Protein werden in der vorliegenden Anmeldung synonym verwendet, da ein Protein ein Polypeptid ist.

Für die Expression eines Gens wesentlich sind Sequenzen mit einer Steuerungsfunktion für die Expression dieses Gens (sog. genregulatorische Sequenzen) wie insbesondere Promotor-Sequenzen, aber auch beispielsweise Enhancer-Sequenzen oder allgemein weitere genregulatorische Sequenzen. Häufig befinden sich solche Sequenzen in benachbarten Sequenzabschnitten zu dem für das Polypeptid codierenden Nukleinsäureabschnitt, den sogenannten flankierenden Bereichen. Die flankierenden Bereiche können dabei ausgehend von den bekannten Sequenzen über an sich bekannte Methoden, beispielsweise mithilfe nach außen gerichteter PCR-Primer und einer Präparation genomischer DNA als Matrize ermittelt werden ("anchored PCR"). Beispielsweise wird hierbei von einer Präparation der genomischen DNA des Organismus ausgegangen, dessen Nukleinsäure verwendet werden soll. Dabei dient eine bekannte DNA-Sequenz, beispielsweise ein Teil der für ein Polypeptid codierenden Sequenz, als Sonde zur Identifizierung gentragender Fragmente in einer Southern-Blot-Hybridisierung. Positive Fragmente können nach bekannten und im Stand der Technik etablierten Methoden isoliert und einschließlich der flankierenden Bereiche in Vektoren kloniert werden. Anhand derselben Sequenz können dann nach außen weisende Sequenzier-Primer entworfen und die flankierenden unbekannten Bereiche über Sequenzierung erschlossen werden. Hierbei liegt der Promotorbreich oftmals unmittelbar vor, das heißt 5'-wärts von dem proteincodierenden Teil und umfasst selten mehr als wenige hundert Nukleotide. Alternativ zu diesem experimentellen Ansatz können derartige nichtcodierende Bereiche zumindest zum Teil für viele Gene aus Datenbankeinträgen für die jeweiligen Mikroorganismen oder für mit diesen verwandte Spezies entnommen werden, beispielsweise der Datenbank SubtiList des Institute Pasteur, Paris, Frankreich (http://genolist.pasteur.fr/SubtiList/index.html ), insbesondere für Bacillus subtilis.

Unter einem Promotor wird demnach eine DNA-Sequenz verstanden, die die regulierte Expression eines Gens ermöglicht. Natürlicherweise ist eine Promotorsequenz ein Bestandteil eines Gens und liegt oftmals an dessen 5'-Ende und somit vor dem RNAkodierenden Bereich. Die wichtigste Eigenschaft eines Promotors ist die spezifische Wechselwirkung mit mindestens einem DNA-bindenden Protein bzw. Polypeptid, welches den Start der Transkription des Gens durch eine RNA-Polymerase vermittelt und als Transkriptionsfaktor bezeichnet wird. Häufig sind mehrere Transkriptionsfaktoren und/oder weitere Proteine am Start der Transkription durch eine RNA-Polymerase beteiligt. Ein Promotor ist demnach eine DNA-Sequenz mit Promotoraktivität, d. h. eine DNA-Sequenz, an die mindestens ein Transkriptionsfaktor zur Initiation der Transkription eines Gens zumindest transient bindet. Die Stärke eines Promotors ist messbar über die Transkriptionshäufigkeit des exprimierten Gens, also über die Anzahl der pro Zeiteinheit erzeugten RNA-Moleküle, insbesondere mRNA-Moleküle.

Wichtige bakterielle Transkriptionsfaktoren sind die Sigma-Faktoren. Die Struktur eines bakteriellen Promotors ist daher vor allem davon abhängig, von welchem der Sigma-Faktoren er erkannt wird. Bei dem Bakterien-Modellorganismus Escherichia coli (E. coli) werden die meisten Promotoren über den Faktor Sigma-70 erkannt. Der zu E. coli Sigma-70 analoge Faktor in Bacillus, beispielsweise Bacillus subtilis, ist Sigma-A (SigA).

Anhand von Konsensussequenzen lassen sich bei Genen folgende allgemeine Promotorelemente klassifizieren:
- eine -35-Region mit der Konsensussequenz: 5'-TTGACA-3' (SEQ ID NO.1),
- eine -10-Region (Pribnow-Box oder Pribnow-Schaller-Box) mit der Konsensussequenz: 5'-TATAAT-3' (SEQ ID NO. 2),
- ein AT-basenpaarreiches "upstream"-Element (stromaufwärts) oberhalb der -35 und/oder - 10 Region.

Betreffend die Nomenklatur der Positionen erhält das erste transkribierte Nukleotid bzw. Basenpaar auf DNA-Ebene die Position +1, so dass die Positionen weiter stromabwärts (5') liegender Nukleotide bzw. Basenpaare negative Vorzeichen erhalten, während weiter stromaufwärts (3') liegende Nukleotide bzw. Basenpaare mit weiter positiven Positionen (+2, +3, +4, usw.) bezeichnet werden.

Während die -35-Region und -10 Region hauptsächlich durch einen Sigma-Faktor erkannt werden, ist das "upstream"-Element oftmals befähigt, mit der α-Untereinheit einer bakteriellen RNA-Polymerase direkt zu interagieren.

Die Konsensussequenzen sind jedoch nur ein ungefährer Anhaltspunkt für den Aufbau eines Promotors. Spezifische Promotoren können an einer oder mehreren Stellen von diesen Sequenzen abweichen.

Ferner sind starke Promotoren direkt vor dem Startpunkt der Transkription häufig reich an AT-Basenpaaren, was das für die Transkription notwendige Entwinden der DNA-Doppelhelix vereinfacht, da AT-Basenpaare weniger Wasserstoffbrücken als GC-Basenpaare ausbilden.

Der Promotor des Gens subC (subC Promotor) codierend für ein Subtilisin ist eine DNA-Sequenz mit Promotoraktivität, welche in einem Bakterium der Gattung Bacillus natürlicherweise die Transkription eines Gens codierend für ein Subtilisin bewirkt bzw. für diese notwendig ist. Eine für subC-Promotor ebenfalls gebräuchliche Bezeichnung ist aprE-Promotor. Beispielsweise wird das entsprechende für ein Subtilisin codierende Gen in Bacillus subtilis in der Regel als aprE bezeichnet, wohingegen das entsprechende für ein Subtilisin codierende Gen in Bacillus licheniformis in der Regel als subC bezeichnet wird, welches für Subtilisin Carlsberg codiert. Natürlicherweise bedeutet in diesem Zusammenhang, dass das Subtilisin ein bakterieneigenes Polypeptid bzw. Protein ist, dessen Gen von dem Bakterium in seiner Wildtyp-Form transkribiert wird.

Bevorzugt umfasst ein erfindungsgemäßer subC-Promotor eine -35- und eine -10 Region, die durch eine Spacer-Sequenz getrennt sind. Bevorzugt weist der Promotor eine -10 Region auf, die eine hohe Ähnlichkeit zu der Konsensus-Sequenz aufweist, beispielsweise 5'-TAcAAT-3' (SEQ ID NO. 3). Die -35 Region kann größere Abweichungen von der Konsensus-Sequenz aufweisen und beispielsweise in nur zwei der sechs Nukleotide mit der Konsensus-Sequenz übereinstimmen, d. h. beispielsweise 5'-TactaA-3' (SEQ ID NO.4). Betreffend die Nomenklatur stellen Kleinbuchstaben von der Konsensus-Sequenz abweichende Nukleotide dar. Auch die Sequenzen um die -35 und -10 Regionen herum, so beispielsweise der Spacer zwischen der -35 und der -10 Region, der beispielsweise 17 Basenpaare lang sein kann, oder die Region 3' (stromaufwärts) der -10 Region (bis zu +40) sind für die Stärke des Promotors von Bedeutung.

Weiter bevorzugt bewirkt der subC-Promotor in einem Bakterium der Gattung Bacillus natürlicherweise die Transkription eines Gens codierend für Subtilisin Carlsberg oder einer Subtilisin Carlsberg entsprechenden Aminosäuresequenz bzw. ist für diese notwendig. Das Subtilisin Carlsberg wird in den Publikationen von E. L. Smith et al. (1968) in J. Biol. Chem., Vol. 243, S. 2184ff und von Jacobs et al. (1985) in Nucl. Acids Res., Band 13, S. 8913ff sowie Jacobs et al. (1995) in Gene 152(1), S. 69ff beschrieben und weist eine Aminosäuresequenz gemäß SEQ ID NO. 5 auf. Es wird natürlicherweise von Bacillus licheniformis gebildet. Besonders bevorzugt handelt es sich bei dem subC-Promotor daher um denjenigen Promotor, der in Bacillus licheniformis natürlicherweise die Transkription des Gens codierend für Subtilisin Carlsberg oder einer Subtilisin Carlsberg entsprechenden Aminosäuresequenz bewirkt bzw. für diese notwendig ist. Eine Subtilisin Carlsberg entsprechende Aminosäuresequenz ist zu derjenigen von Subtilisin Carlsberg zunehmend bevorzugt zu 50%, 55%, 60%, 65%, 70%, 75%, 80%, 85%, 87,5%, 90%, 92,5%, 95%, 97,5%, 98%, 98,5%, 99% und 99,5% identisch.

Ein erfindungsgemäß weiter bevorzugter subC-Promotor ist offenbart als "ATCC 53926 alkaline protease gene promotor" in der internationalen Patentanmeldung WO 91/02792. Besonders bevorzugt umfasst ein erfindungsgemäßer subC-Promotor eine Sequenz, die der in Figur 27 in WO 91/02792 offenbarten Sequenz in den Positionen 84 bis zur Transkriptionsstartstelle ("transcription start site") entspricht (angegeben als SEQ ID NO.7), oder eine Sequenz, die zu dieser zu mindestens 75% identisch ist.

Der Promotor des Gens rpsB (rpsB Promotor) codierend für ein ribosomales Protein S2 ist eine DNA-Sequenz mit Promotoraktivität, welche in einem Bakterium der Gattung Bacillus natürlicherweise die Transkription eines Gens codierend für das ribosomale Proteins S2 bewirkt bzw. für diese notwendig ist.

Ein ribosomales Protein ist eines der Proteine, die in Verbindung mit ribosomaler RNA (rRNA) und weiteren Proteinen eine ribosomale Untereinheit eines Ribosoms bilden. Ein bakterielles Ribosom umfasst ca. 22 Proteine in der kleinen Untereinheit (Proteine S1 bis S22) und ca. 34 Proteine in der großen Untereinheit (Proteine L1 bis L36). Ein ribosomales Protein wird in beiden Untereinheiten gefunden (S20 bzw. L26 sind daher identisch), L7 und L12 sind acetylierte und methylierte Formen desselben Proteins, und L8 ist ein Komplex von L7/L12 und L10. Außerdem existiert L31 in zwei Formen, nämlich als Gesamtlängenprotein (7.9 Kilodalton (kDa) und in verkürzter Form (7.0 kDa). Daher sind in einem bakteriellen Ribosom insgesamt 56 Proteine enthalten. Mit Ausnahme von S1 (mit einem Molekulargewicht von 61.2 kDa) weisen die anderen ribosomalen Proteine Molekulargewichte zwischen 4.4 und 29.7 kDa auf. In der kleinen ribosomalen Untereinheit binden die Proteine S4, S7, S8, S15, S17 und S20 unabhängig voneinander an die 16S rRNA. Nach dem Zusammenbau dieser primären Einheit binden S5, S6, S9, S12, S13, S16, S18, und S19 an das wachsende Ribosom. Diese Proteine unterstützen auch die Anlagerung von S2, S3, S10, S11, S14 und S21. Die ribosomalen Proteine enthalten in der Regel kompakte, globuläre Domänen. Ferner enthalten sie auch lange Abschnitte, die mit der rRNA Wechselwirken können. Das ribosomale Protein S2 ist damit ein Bestandteil der kleinen ribosomalen Untereinheit eines bakteriellen Ribosoms.

Besonders bevorzugt bewirkt der rpsB Promotor in Bacillus subtilis natürlicherweise die Transkription eines Gens codierend für das ribosomale Proteins S2 von Bacillus subtilis bzw. ist für diese notwendig. Das ribosomale Protein S2 von Bacillus subtilis ist beispielsweise verfügbar unter dem Datenbankeintrag P21464 der öffentlich zugänglichen Datenbank UniProtKB/Swiss-Prot (EMBL Gutstation, European Bioinformatics Institute (EBI), Wellcome Trust Genome Campus, Hinxton Cambridge CB10 1SD, United Kingdom).

Neben den beiden Promotoren umfasst eine erfindungsgemäße Nukleinsäure eine Nukleinsäuresequenz, die für ein Polypeptid codiert. Hierfür ist grundsätzlich eine beliebige Nukleinsäuresequenz verwendbar, die in ein Polypeptid translatiert werden kann.

Die erste Promotorsequenz und die zweite Promotorsequenz liegen hintereinander auf dem Nukleinsäuremolekül vor. Die zweite Promotorsequenz befindet sich vor der ersten Promotorsequenz auf dem Nukleinsäuremolekül (in 5' → 3'-Orientierung). Bevorzugt befinden sich zwischen den beiden Promotorsequenzen (a) und (b) keine Nukleinsäuresequenzen, die die Transkriptionshäufigkeit der für das Polypeptid codierenden Nukleinsäuresequenz (c) vermindern. Ferner liegen zwischen den beiden Promotorsequenzen (a) und (b) maximal 500 und zunehmend bevorzugt maximal 450, 425, 400, 375, 350, 325, 300, 275, 250, 225, 200, 175, 150, 125, 100, 90, 80, 70, 60, 50, 40, 30, 20, 15, 14, 13, 12, 11, 10, 9, 8, 7, 6 oder 5 Nukleotide. Besonders bevorzugt liegen die beiden Promotorsequenzen auf dem Nukleinsäuremolekül vor der für das Polypeptid codierenden Nukleinsäuresequenz (c) in 5' → 3'-Orientierung. Ganz besonders bevorzugt liegen ferner zwischen der ersten Promotorsequenz (a) und der für das Polypeptid codierenden Nukleinsäuresequenz (c) maximal 500 und zunehmend bevorzugt maximal 450, 425, 400, 375, 350, 325, 300, 275, 250, 225, 200, 175, 150, 125, 100, 90, 80, 70, 60, 50, 40, 30, 20, 15, 14, 13, 12, 11, 10, 9, 8, 7, 6 oder 5 Nukleotide. Alle vorstehenden Angaben beziehen sich auf denjenigen DNA-Strang, der die für das Polypeptid codierende Nukleinsäuresequenz (c) beinhaltet und nicht auf den zugehörigen codogenen DNA-Strang. Ausgehend von der für das Polypeptid codierenden Nukleinsäuresequenz (c) befinden sich die beiden Promotorsequenzen damit weiter stromabwärts, d. h. in 3'-Richtung, auf diesem DNA-Strang mit den vorstehend genannten Maßgaben.

Die erfindungsgemäße Nukleinsäure ist dadurch gekennzeichnet, dass sie eine nicht natürliche Nukleinsäure ist. Nicht natürlich bedeutet, dass eine erfindungsgemäße Nukleinsäure nicht aus einem in der Natur vorkommenden Organismus in seiner Wildtyp-Form isoliert werden kann. Insbesondere und bezogen auf Wildtyp-Bakterien ist eine erfindungsgemäße Nukleinsäure daher keine bakterieneigene Nukleinsäure. Vielmehr ist eine erfindungsgemäße Nukleinsäure das Ergebnis einer gentechnischen Modifikation, d. h. eine erfindungsgemäße Nukleinsäure wurde mit Hilfe gentechnologischer Verfahren erzeugt durch Kombination der Gensequenzen (a) und/oder (b) und/oder (c). In der Natur vorkommende Nukleinsäuren, beispielsweise genomische DNA von Bakterien, sind daher nicht Gegenstand der Erfindung.

Bevorzugt stammen die Sequenzen (a) und/oder (b) und/oder (c) nicht von demselben bzw. denselben Organsimen, insbesondere Bakterien, sondern stammen von unterschiedlichen Organismen, insbesondere Bakterien. Unterschiedliche Bakterien sind beispielsweise Bakterien, die unterschiedlichen Stämmen oder Arten oder Gattungen angehören.

In einer weiteren Ausführungsform der Erfindung ist eine erfindungsgemäße Nukleinsäure dadurch gekennzeichnet, dass die erste Promotorsequenz und die zweite Promotorsequenz derart funktionell gekoppelt sind, dass sie eine verstärkte Expression der Nukleinsäuresequenz c) in einer Wirtszelle bewirken im Vergleich mit einer Wirtszelle, in der die Expression der Nukleinsäuresequenz c) mit nur einer der Promotorsequenzen a) oder b) erfolgt.

Dieser Sachverhalt wird ermittelt über die Transkriptionshäufigkeit der für das Polypeptid codierenden Nukleinsäuresequenz (c), also über die Anzahl der pro Zeiteinheit erzeugten RNA-Moleküle. Die gemeinsame Promotoraktivität der ersten und zweiten Promotorsequenzen übersteigt demnach die Promotoraktivität der ersten Promotorsequenz bzw. der zweiten Promotorsequenz. Genauso kann verglichen werden, wie viele entsprechende Polypeptide pro Zeiteinheit gebildet werden. Im letztgenannten Fall wird daher der Translationsschritt in die Bestimmung der Expression mit einbezogen. Bei einer erfindungsgemäßen Nukleinsäure bewirken die beiden Promotoren daher eine Steigerung der Expression der für ein Polypeptid codierenden Nukleinsäuresequenz und damit, dass diese Nukleinsäuresequenz häufiger in RNA übersetzt wird als bei einer Vergleichsnukleinsäure, die zwar die für das Polypeptid codierende Nukleinsäuresequenz umfasst, aber nur einen der beiden Promotoren. Bei der Vergleichsnukleinsäure bewirkt der Promotor daher ebenfalls eine Expression der für ein Polypeptid codierenden Nukleinsäuresequenz, jedoch wird diese Nukleinsäuresequenz weniger häufiger in RNA übersetzt als bei einer erfindungsgemäßen Nukleinsäure. Ein solcher Vergleich erfolgt erfindungsgemäß in Wirtszellen gleichen Typs, um die Vergleichbarkeit der Messungen zu gewährleisten. Bevorzugt wird die funktionelle Kopplung dadurch erreicht, dass die erste Promotorsequenz (a) und/oder die zweite Promotorsequenz (b) und/oder die für das Polypeptid codierende Nukleinsäuresequenz (c) so auf dem bzw. innerhalb des Nukleinsäuremoleküls angeordnet sind, wie es vorstehend beschrieben ist.

Erfindungsgemäße Nukleinsäuren können über an sich bekannte Verfahren zur Veränderung von Nukleinsäuren erzeugt werden. Solche sind beispielsweise in einschlägigen Handbüchern wie dem von Fritsch, Sambrook und Maniatis, "Molecular cloning: a laboratory manual", Cold Spring Harbour Laborstory Press, New York, 1989, dargestellt. Beispiele für solche Verfahren sind die chemische Synthese oder die Polymerase-Kettenreaktion (PCR), optional in Verbindung mit weiteren molekularbiologischen und/oder chemischen bzw. biochemischen Standardmethoden.

In einer gesonderten Ausführungsform der Erfindung ist die Nukleinsäure dadurch gekennzeichnet, dass der Promotor des Gens subC codierend für Subtilisin Carlsberg natürlicherweise in Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans oder Bacillus pumilus vorhanden ist und/oder der Promotor des Gens rpsB codierend für das ribosomale Protein S2 natürlicherweise in Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans oder Bacillus pumilus vorhanden ist.

In einer weiteren Ausführungsform ist die Nukleinsäure dadurch gekennzeichnet, dass die zweite Promotorsequenz b) eine Nukleinsäuresequenz umfasst, die zu SEQ ID NO. 6 in den Positionen 7 bis 234 zunehmend bevorzugt zu mindestens 78,5%, 80%, 82,5%, 85%, 87,5%, 90%, 91%, 92%, 93%, 93,5%, 94%, 94,5%, 95%, 95,5%, 96%, 96,5%, 97%, 97,5%, 98%, 98,5%, 99% und ganz besonders bevorzugt zu 100% identisch ist.

Es hat sich herausgestellt, dass solche Promotoren besonders geeignet sind, eine hohe Expression der für ein Polypeptid codierenden Nukleinsäuresequenz und damit eine hohe Produktausbeute zu bewirken.

Die Bestimmung der Identität erfolgt durch einen Sequenzvergleich. Solch ein Vergleich geschieht dadurch, dass ähnliche Abfolgen in den Nukleotidsequenzen (oder auch Aminosäuresequenzen) einander zugeordnet werden. Eine tabellarische Zuordnung der betreffenden Positionen wird als Alignment bezeichnet. Bei der Analyse von Nukleotidsequenzen sind wiederum beide komplementären Stränge und jeweils allen drei möglichen Leserastern zu berücksichtigen; ebenso die Degeneriertheit des genetischen Codes und die organismenspezifische Verwendung der Codons (Codon-Usage). Inzwischen werden Alignments über Computerprogramme erstellt, wie beispielsweise durch die Algorithmen FASTA oder BLAST; dieses Vorgehen wird beispielsweise von D. J. Lipman und W. R. Pearson (1985) in Science, Band 227, S. 1435-1441 beschrieben.

Eine Zusammenstellung aller in den verglichenen Sequenzen übereinstimmenden Positionen wird als Konsensus-Sequenz bezeichnet.

Solch ein Vergleich erlaubt auch eine Aussage über die Ähnlichkeit oder Homologie der verglichenen Sequenzen zueinander. Diese wird in Prozent Identität, das heißt dem Anteil der identischen Nukleotide oder Aminosäurereste an denselben bzw. in einem Alignment einander entsprechenden Positionen wiedergegeben. Ein weiter gefasster Homologiebegriff bezieht bei Aminosäuresequenzen konservierte Aminosäure-Austausche in diesen Wert mit ein. Es ist dann von Prozent Ähnlichkeit die Rede. Solche Aussagen können über ganze Polypeptide oder Gene oder nur über einzelne Bereiche getroffen werden.

Homologe oder identische Bereiche von verschiedenen Nukleinsäure- oder Aminosäuresequenzen sind durch Übereinstimmungen in den Sequenzen definiert. Diese können auch durch identische Funktion gekennzeichnet sein. Sie geht bis zu völligen Identitäten in kleinsten Bereichen, sogenannten Boxen, die nur wenige Nukleotide bzw. Aminosäuren umfassen und meist für die Gesamtaktivität essentielle Funktionen ausüben. Beispiele für solche Funktionen können bei Nukleinsäuren die Wechselwirkung mit speziellen Molekülen sein, beispielsweise bei Promotorsequenzen die Wechselwirkung mit bestimmten Transkriptionsfaktoren. Bei Aminosäuresequenzen können solche Funktionen auch kleinste Teilfunktionen der vom gesamten Polypeptid ausgeübten Funktion verstanden werden wie beispielsweise die Ausbildung einzelner Wasserstoffbrückenbindungen zur Komplexierung eines Substrats oder Übergangskomplexes.

In einer weiteren Ausführungsform der Erfinundung ist eine erfindungsgemäße Nukleinsäure dadurch gekennzeichnet, dass die Nukleinsäuresequenz c) für ein Enzym codiert, insbesondere für eine Protease, Amylase, Cellulase, Hemicellulase, Mannanase, Tannase, Xylanase, Xanthanase, β-Glucosidase, Carrageenase, Oxidase, Oxidoreduktase oder Lipase. Solche Enzyme werden bevorzugt mit einer erfindungsgemäßen Nukleinsäure hergestellt. Besonders bevorzugte Enzyme, die mit einer erfindungsgemäßen Nukleinsäure hergestellt werden können, werden nachstehend beschrieben. Für alle genannten Moleküle ist deren Nukleinsäuresequenz entweder im Stand der Technik offenbart oder über fachübliche Routinemaßnahmen der Sequenzierung eindeutig bestimmbar, so dass der Fachmann eine Sequenz erhält, die er als Nukleinsäuresequenz c) in eine erfindungsgemäße Nukleinsäure einbringen kann. Beispielsweise kann der Fachmann ausgehend von einem Handelsprodukt, in diesem Fall einem Polypeptid, dessen Aminosäuresequenz ermitteln und diese wiederum unter Zugrundelegung des genetischen Codes in eine Nukleinsäuresequenz übersetzen.

Unter den Proteasen sind solche vom Subtilisin-Typ bevorzugt. Beispiele hierfür sind die Subtilisine BPN' und Carlsberg, die Protease P892, die Subtilisine 147 und 309, die Alkalische Protease aus Bacillus lentus, Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase® von der Firma Novozymes A/S, Bagsvaerd, Dänemark, erhältlich. Die Subtilisine 147 und 309 werden unter den Handelsnamen Esperase®, beziehungsweise Savinase® von der Firma Novozymes vertrieben. Von der Protease aus Bacillus lentus DSM 5483 leiten sich die unter der Bezeichnung BLAP® geführten Protease-Varianten ab. Weitere brauchbare Proteasen sind beispielsweise die unter den Handelsnamen Durazym®, Relase®, Everlase®, Nafizym®, Natalase®, Kannase® und Ovozyme® von der Firma Novozymes, die unter den Handelsnamen, Purafect®, Purafect® OxP, Purafect® Prime, Excellase® und Properase® von der Firma Genencor, das unter dem Handelsnamen Protosol® von der Firma Advanced Biochemicals Ltd., Thane, Indien, das unter dem Handelsnamen Wuxi® von der Firma Wuxi Snyder Bioproducts Ltd., China, die unter den Handelsnamen Proleather® und Protease P® von der Firma Amano Pharmaceuticals Ltd., Nagoya, Japan, und das unter der Bezeichnung Proteinase K-16 von der Firma Kao Corp., Tokyo, Japan, erhältlichen Enzyme. Besonders bevorzugt eingesetzt werden auch die Proteasen aus Bacillus gibsonii und Bacillus pumilus, die offenbart sind in den internationalen Patentanmeldungen WO 2008/086916 und WO 2007/131656.

Beispiele für Amylasen sind die α-Amylasen aus Bacillus licheniformis, aus B. amyloliquefaciens oder aus B. stearothermophilus sowie deren für den Einsatz in Wasch- oder Reinigungsmitteln verbesserte Weiterentwicklungen. Das Enzym aus B. licheniformis ist von der Firma Novozymes unter dem Namen Termamyl® und von der Firma Genencor unter dem Namen Purastar®ST erhältlich. Weiterentwicklungsprodukte dieser α-Amylase sind von der Firma Novozymes unter den Handelsnamen Duramyl® und Termamyl®ultra, von der Firma Genencor unter dem Namen Purastar®OxAm und von der Firma Daiwa Seiko Inc., Tokyo, Japan, als Keistase® erhältlich. Die α-Amylase von B. amyloliquefaciens wird von der Firma Novozymes unter dem Namen BAN® vertrieben, und abgeleitete Varianten von der α-Amylase aus B. stearothermophilus unter den Namen BSG® und Novamyl®, ebenfalls von der Firma Novozymes.

Des Weiteren sind für diesen Zweck die α-Amylase aus Bacillus sp. A 7-7 (DSM 12368) und die Cyclodextrin-Glucanotransferase (CGTase) aus B. agaradherens (DSM 9948) hervorzuheben. Ferner sind die amylolytischen Enzyme einsetzbar, die dem Sequenzraum von α-Amylasen angehören, der in der internationalen Patentanmeldung WO 03/002711 A2 definiert wird, und die, die in der Anmeldung WO 03/054177 A2 beschrieben werden. Ebenso sind Fusionsprodukte der genannten Moleküle einsetzbar.

Darüber hinaus sind die unter den Handelsnamen Fungamyl® von der Firma Novozymes erhältlichen Weiterentwicklungen der α-Amylase aus Aspergillus niger und A. oryzae geeignet. Weitere einsetzbare Handelsprodukte sind beispielsweise die Amylase-LT® und Stainzyme® bzw. Stainzyme ultra® oder Stainzyme plus®, letztere ebenfalls von der Firma Novozymes. Auch durch Punktmutationen erhältliche Varianten dieser Enzyme können erfindungsgemäß eingesetzt werden.

Beispiele für Lipasen oder Cutinasen, die insbesondere wegen ihrer Triglycerid-spaltenden Aktivitäten enthalten sind, aber auch, um aus geeigneten Vorstufen in situ Persäuren zu erzeugen, sind die ursprünglich aus Humicola lanuginosa (Thermomyces lanuginosus) erhältlichen, beziehungsweise weiterentwickelten Lipasen, insbesondere solche mit dem Aminosäureaustausch D96L. Sie werden beispielsweise von der Firma Novozymes unter den Handelsnamen Lipolase®, Lipolase®Ultra, LipoPrime®, Lipozyme® und Lipex® vertrieben. Desweiteren sind beispielsweise die Cutinasen einsetzbar, die ursprünglich aus Fusarium solani pisi und Humicola insolens isoliert worden sind. Ebenso brauchbare Lipasen sind von der Firma Amano unter den Bezeichnungen Lipase CE®, Lipase P®, Lipase B® beziehungsweise Lipase CES®, Lipase AKG®, Bacills sp. Lipase®, Lipase AP®, Lipase M-AP® und Lipase AML® erhältlich. Von der Firma Genencor sind beispielsweise die Lipasen beziehungsweise Cutinasen einsetzbar, deren Ausgangsenzyme ursprünglich aus Pseudomonas mendocina und Fusarium solanii isoliert worden sind. Als weitere wichtige Handelsprodukte sind die ursprünglich von der Firma Gist-Brocades vertriebenen Präparationen M1 Lipase® und Lipomax® und die von der Firma Meito Sangyo KK, Japan, unter den Namen Lipase MY-30®, Lipase OF® und Lipase PL® vertriebenen Enzyme zu erwähnen, ferner das Produkt Lumafast® von der Firma Genencor.

Beispiele für Cellulasen umfassen eine Endoglucanase(EG)-reiche Cellulase-Präparation, beziehungsweise deren Weiterentwicklungen, die von der Firma Novozymes unter dem Handelsnamen Celluzyme® angeboten wird. Die ebenfalls von der Firma Novozymes erhältlichen Produkte Endolase® und Carezyme® basieren auf der 50 kD-EG, beziehungsweise der 43 kD-EG aus H. insolens DSM 1800. Weitere einsetzbare Handelsprodukte dieser Firma sind Cellusoft®, Renozyme® und Cellucean®. Weiterhin einsetzbar sind beispielsweise die 20 kD-EG aus Melanocarpus, die von der Firma AB Enzymes, Finnland, unter den Handelsnamen Ecostone® und Biotouch® erhältlich sind. Weitere Handelsprodukte der Firma AB Enzymes sind Econase® und Ecopulp®. Weitere geeignete Cellulasen sind aus Bacillus sp. CBS 670.93 und CBS 669.93, wobei die aus Bacillus sp. CBS 670.93 von der Firma Genencor unter dem Handelsnamen Puradax® erhältlich ist. Weitere Handelsprodukte der Firma Genencor sind "Genencor detergent cellulase L" und IndiAge®Neutra.

Zu Hemicellulasen zählen beispielsweise Mannanasen, Xanthanlyasen (Xanthanasen), Pektinlyasen (=Pektinasen), Pektinesterasen, Pektatlyasen, Xyloglucanasen (=Xylanasen), Pullulanasen und β-Glucanasen. Diesbezüglich geeignete Enzyme sind beispielsweise unter den Namen Gamanase® und Pektinex AR® von der Firma Novozymes, unter dem Namen Rohapec® B1 L von der Firma AB Enzymes und unter dem Namen Pyrolase® von der Firma Diversa Corp., San Diego, CA, USA erhältlich. Die aus Bacillus subtilis gewonnene β-Glucanase ist unter dem Namen Cereflo® von der Firma Novozymes erhältlich. Erfindungsgemäß besonders bevorzugte Hemicellulasen sind Mannanasen, welche beispielsweise unter den Handelsnamen Mannaway(R) von dem Unternehmen Novozymes oder Purabrite® von dem Unternehmen Genencor vertrieben werden.

Oxidoreduktasen umfassen beispielsweise Oxidasen, Oxygenasen, Katalasen (die bei niedrigen H₂O₂-Konzentrationen als Peroxidase reagieren), Peroxidasen, wie Halo-, Chloro-, Bromo-, Lignin-, Glucose- oder Manganperoxidasen, Dioxygenasen oder Laccasen (Phenoloxidasen, Polyphenoloxidasen). Als geeignete Handelsprodukte sind Denilite® 1 und 2 der Firma Novozymes zu nennen. Weitere Beispiele für Oxidasen sind als Komponenten für eine enzymatische Perhydrolyse in den internationalen Patentanmeldungen WO 98/45398 A1, WO 2005/056782 A2 sowie WO 2004/058961 A1 offenbart. Ein kombiniertes enzymatisches Bleichsystem, umfassend eine Oxidase und eine Perhydrolase beschreibt die Anmeldung WO 2005/124012.

Die Nukleinsäuren, die für erfindungsgemäß bevorzugte Enzyme codieren, stammen ursprünglich entweder aus Mikroorganismen, etwa der Gattungen Bacillus, Streptomyces, Humicola, Escherichia oder Pseudomonas, und/oder wurden nach an sich bekannten biotechnologischen Verfahren in geeigneten Mikroorganismen rekombinant erhalten, beispielsweise in Bakterien der Gattungen Bacillus oder in filamentösen Fungi.

Mit allen genannten Erfindungsgegenständen und Ausführungsformen sind als weitere Erfindungsgegenstände entsprechende Vektoren, Wirtszellen, Herstellungsverfahren, in denen entsprechende Vektoren oder Wirtszellen eingesetzt werden sowie Verwendungen entsprechender Nukleinsäuren, Vektoren und Wirtszellen verbunden. Daher betreffen die vorstehenden Ausführungen diese Erfindungsgegenstände entsprechend.

Einen weiteren Gegenstand der Erfindung ist somit ein Vektor, der eine erfindungsgemäße Nukleinsäure enthält, insbesondere ein Klonierungsvektor oder ein Expressionsvektor.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen dieses in einer Spezies oder einer Zellinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Man unterscheidet in der Gentechnik einerseits zwischen solchen Vektoren, die der Lagerung und somit gewissermaßen auch der gentechnischen Arbeit dienen, den sogenannten Klonierungsvektoren, und andererseits denen, die die Funktion erfüllen, das interessierende Gen in der Wirtszelle zu realisieren, das heißt, die Expression des betreffenden Polypeptids zu ermöglichen. Diese Vektoren werden als Expressionsvektoren bezeichnet.

Im Rahmen der vorliegenden Erfindung wird eine erfindungsgemäße Nukleinsäure geeigneterweise in einen Vektor kloniert. Dazu können beispielsweise solche gehören, deren Ursprung bakterielle Plasmide, Viren oder Bacteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Es ist dabei im Sinne der Erfindung unerheblich, ob sie extrachomosomal als eigene Einheiten vorliegen oder in ein Chromosom bzw. chromosomale DNA integrieren. Welches der zahlreichen aus dem Stand der Technik bekannten Systeme gewählt wird, hängt vom Einzelfall ab. Ausschlaggebend können beispielsweise die erreichbare Kopienzahl, die zur Verfügung stehenden Selektionssysteme, darunter vor allem Antibiotikaresistenzen, oder die Kultivierbarkeit der zur Aufnahme der Vektoren befähigten Wirtszellen sein.

Expressionsvektoren umfassen Teilsequenzen, die sie dazu befähigen, in den sie enthaltenden Wirtszellen, vorzugsweise Mikroorganismen, besonders bevorzugt Bakterien, zu replizieren und dort das enthaltene Gen zur Expression zu bringen. Die Expression wird insbesondere von den Promotoren beeinflusst, welche die Transkription des Gens regulieren. Prinzipiell kann die Expression durch den natürlichen, ursprünglich vor dem zu exprimierenden Gen lokalisierten Promotor erfolgen, aber auch nach gentechnischer Fusion sowohl durch einen auf dem Expressionsvektor bereitgestellten Promotor der Wirtszelle als auch durch einen modifizierten oder einen völlig anderen Promotor eines anderen Organismus oder einer anderen Wirtszelle. Im vorliegenden Fall werden zumindest zwei Promotoren bereits durch eine erfindungsgemäße Nukleinsäure zur Verfügung gestellt und für die Expression der Nukleinsäuresequenz c) genutzt.

Expressionsvektoren können über Änderungen der Kulturbedingungen oder Zugabe von bestimmten Verbindungen, wie beispielsweise die Zelldichte oder spezielle Faktoren, regulierbar sein. Expressionsvektoren ermöglichen, dass das zugehörige Polypeptid heterolog, also in einer anderen Zelle bzw. Wirtszelle als derjenigen, aus der es natürlicherweise gewonnen werden kann, produziert wird. Die Zellen können dabei zu verschiedenen Organismen zugehörig sein oder von verschiedenen Organismen stammen. Auch eine homologe Gewinnung des Polypeptids aus einer das Gen natürlicherweise exprimierenden Wirtszelle über einen erfindungsgemäßen Vektor ist möglich. Dies kann den Vorteil aufweisen, dass natürliche, mit der Translation in einem Zusammenhang stehende Modifikationsreaktionen an dem entstehenden Polypeptid genauso durchgeführt werden, wie sie auch natürlicherweise ablaufen würden.

Zu einem einsetzbaren Expressionssystem können ferner zusätzliche Gene zählen, beispielsweise solche, die auf anderen Vektoren zur Verfügung gestellt werden, und die die Produktion erfindungsgemäßer Polypeptide beeinflussen. Hierbei kann es sich um modifizierende Genprodukte handeln oder um solche, die mit dem Polypeptid, das von der Nukleinsäuresequenz c) codiert wird, gemeinsam aufgereinigt werden sollen, etwa um dessen enzymatische Funktion zu beeinflussen. Dabei kann es sich beispielsweise um andere Proteine oder Enzyme, um Inhibitoren oder um solche Elemente handeln, die die Wechselwirkung mit verschiedenen Substraten beeinflussen.

Ein weiterer Gegenstand der Erfindung ist eine nicht menschliche Wirtszelle, die eine erfindungsgemäße Nukleinsäure oder einen erfindungsgemäßen Vektor beinhaltet. Eine erfindungsgemäße Nukleinsäure bzw. ein erfindungsgemäßer Vektor wird bevorzugt in die Wirtszelle eingebracht durch deren Transformation. Erfindungsgemäß bevorzugt erfolgt dieses dadurch, dass eine erfindungsgemäße Nukleinsäure bzw. ein erfindungsgemäßer Vektor in einen Mikroorganismus transformiert wird, der dann eine erfindungsgemäße Wirtszelle darstellt. Alternativ können auch einzelne Komponenten, d. h. Nukleinsäure-Teile bzw. -Fragmente, beispielsweise die Komponenten (a) und/oder (b) und/oder (c) einer erfindungsgemäßen Nukleinsäure, derart in eine Wirtszelle eingebracht werden, dass die dann resultierende Wirtszelle eine erfindungsgemäße Nukleinsäure bzw. einen erfindungsgemäßen Vektor enthält. Dieses Vorgehen eignet sich besonders dann, wenn die Wirtszelle bereits eine oder mehrere Bestandteile einer erfindungsgemäßen Nukleinsäure bzw. eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, das heißt prokaryotische oder eukaryotische Zellen. Bevorzugt sind solche Wirtszellen, die sich genetisch vorteilhaft handhaben lassen, was beispielsweise die Transformation mit der Nukleinsäure oder dem Vektor und dessen stabile Etablierung angeht, beispielsweise einzellige Pilze oder Bakterien. Ferner zeichnen sich bevorzugte Wirtszellen durch eine gute mikrobiologische und biotechnologische Handhabbarkeit aus. Das betrifft beispielsweise leichte Kultivierbarkeit, hohe Wachstumsraten, geringe Anforderungen an Fermentationsmedien und gute Produktions- und Sekretionsraten für Fremdproteine. Häufig müssen aus der Fülle an verschiedenen im Stand der Technik zur Verfügung stehenden Systemen die optimalen Expressionssysteme für den Einzelfall experimentell ermittelt werden.

Weitere bevorzugte Ausführungsformen stellen solche Wirtszellen dar, die aufgrund genetischer Regulationselemente, die beispielsweise auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Polypeptide.

Bevorzugte Wirtszellen sind prokaryontische oder bakterielle Zellen. Bakterien zeichnen sich durch kurze Generationszeiten und geringe Ansprüche an die Kultivierungsbedingungen aus. Dadurch können kostengünstige Verfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein.

Bei gramnegativen Bakterien wie beispielsweise Escherichia coli wird eine Vielzahl von Polypeptiden in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Polypeptide nicht nur in den periplasmatischen Raum, sondern in das das Bakterium umgebende Medium ausschleusen. Grampositive Bakterien wie beispielsweise Bacilli oder Actinomyceten oder andere Vertreter der Actinomycetales besitzen demgegenüber keine äußere Membran, so dass sezernierte Polypeptide sogleich in das die Bakterien umgebende Medium, in der Regel das Nährmedium, abgegeben werden, aus welchem sich die exprimierten Polypeptide aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Usage verfügen und ihr Protein-Syntheseapparat naturgemäß entsprechend ausgerichtet ist.

Unter Codon-Usage wird die Übersetzung des genetischen Codes in Aminosäuren verstanden, d. h. welche Nukleotidfolge (Triplett oder Basentriplett) für welche Aminosäure bzw. für welche Funktion, beispielsweise Beginn und Ende des zu translatierenden Bereichs, Bindungsstellen für verschiedene Proteine, usw., kodiert. So besitzt jeder Organismus, insbesondere jeder Produktionsstamm eine bestimmte Codon-Usage. Es kann zu Engpässen in der Proteinbiosynthese kommen, wenn die auf der transgenen Nukleinsäure liegenden Codons in der Wirtszelle einer vergleichsweise geringen Zahl von beladenen tRNAs gegenüberstehen. Synonyme Codons codieren dagegen für dieselben Aminosäuren und können in Abhängigkeit vom Wirt besser translatiert werden. Dieses gegebenenfalls notwendige Umschreiben hängt somit von der Wahl des Expressionssystems ab. Insbesondere bei Proben aus unbekannten, eventuell nicht kultivierbaren Organismen kann eine entsprechende Anpassung notwendig sein.

Die vorliegende Erfindung ist prinzipiell auf alle Mikroorganismen, insbesondere auf alle fermentierbaren Mikroorganismen, besonders bevorzugt auf solche der Gattung Bacillus, anwendbar und führt dazu, dass sich durch den Einsatz solcher Mikroorganismen als Produktionsorganismen eine erhöhte Produktausbeute in einer Fermentation verwirklichen lässt. Solche Mikroorganismen stellen dann Wirtszellen im Sinne der Erfindung dar.

In einer weiteren Ausführungsform der Erfindung ist die Wirtszelle daher dadurch gekennzeichnet, dass sie ein Bakterium ist, bevorzugt eines, das ausgewählt ist aus der Gruppe der Gattungen von Escherichia, Klebsiella, Bacillus, Staphylococcus, Corynebakterium, Arthrobacter, Streptomyces, Stenotrophomonas und Pseudomonas, weiter bevorzugt eines, das ausgewählt ist aus der Gruppe von Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor und Stenotrophomonas maltophilia.

Die Wirtszelle kann aber auch eine eukaryontische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt.

Im Gegensatz zu prokaryontischen Zellen sind eukaryontische Zellen in der Lage, das gebildete Polypeptid posttranslational zu modifizieren. Beispiele dafür sind Pilze wie Actinomyceten oder Hefen wie Saccharomyces oder Kluyveromyces. Dies kann beispielsweise dann besonders vorteilhaft sein, wenn die Polypeptide im Zusammenhang mit ihrer Synthese spezifische Modifikationen erfahren sollen, die derartige Systeme ermöglichen. Zu den Modifikationen, die eukaryontische Systeme besonders im Zusammenhang mit der Proteinsynthese durchführen, gehören beispielsweise die Bindung niedermolekularer Verbindungen wie Membrananker oder Oligosaccharide. Derartige Oligosaccharid-Modifikationen können beispielsweise zur Senkung der Allergenizität eines exprimierten Polypeptids wünschenswert sein. Auch eine Coexpression mit den natürlicherweise von derartigen Zellen gebildeten Enzymen, wie beispielsweise Cellulasen, kann vorteilhaft sein. Ferner können sich beispielsweise thermophile pilzliche Expressionssysteme besonders zur Expression temperaturbeständiger Varianten eignen.

Als Produkte, die während der Fermentation gebildet werden, werden erfindungsgemäß Polypeptide bzw. Proteine betrachtet, die von der Nukleinsäuresequenz (c) codiert werden. Bevorzugt handelt es sich hierbei um Enzyme.

Die Wirtszellen können ferner hinsichtlich ihrer Anforderungen an die Kulturbedingungen verän dert sein, andere oder zusätzliche Selektionsmarker aufweisen oder andere oder zusätzliche Proteine exprimieren. Es kann sich insbesondere um solche Wirtszellen handeln, die mehrere Proteine oder Enzyme exprimieren. Bevorzugt sezernieren sie diese in das die Wirtszellen umgebende Medium.

Die erfindungsgemäßen Wirtszellen werden in an sich bekannter Weise kultiviert und fermentiert, beispielsweise in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig Produkt aus dem Medium entnommen werden kann.

Erfindungsgemäße Wirtszellen werden bevorzugt verwendet, um Polypeptide bzw. Proteine herzustellen, die von der Nukleinsäuresequenz (c) codiert werden. Ein weiterer Gegenstand der Erfindung ist daher ein Verfahren zur Herstellung eines Polypeptids umfassend
a) Kultivieren einer erfindungsgemäßen Wirtszelle
b) Isolieren des Polypeptids aus dem Kulturmedium oder aus der Wirtszelle.

Dieser Erfindungsgegenstand umfasst bevorzugt Fermentationsverfahren. Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, beispielsweise des Polypeptids. Alle Fermentationsverfahren, die auf einem entsprechenden Verfahren zur Herstellung eines Polypeptids beruhen, stellen Ausführungsformen dieses Erfindungsgegenstandes dar.

Hierbei müssen die jeweils optimalen Bedingungen für die Herstellungsverfahren, insbesondere die optimalen Kulturbedingungen für die benutzten Wirtszellen, nach dem Wissen des Fachmanns experimentell ermittelt werden, beispielsweise hinsichtlich Fermentationsvolumen und/oder Medienzusammensetzung und/oder Sauerstoffversorgung und/oder Rührergeschwindigkeit.

Fermentationsverfahren, die dadurch gekennzeichnet sind, dass die Fermentation über eine Zulaufstrategie durchgeführt wird, kommen ebenfalls in Betracht. Hierbei werden die Medienbestandteile, die durch die fortlaufende Kultivierung verbraucht werden, zugefüttert; man spricht auch von einer Zufütterungsstrategie. Hierdurch können beträchtliche Steigerungen sowohl in der Zelldichte als auch in der Zellmasse bzw. Trockenmasse und/oder vor allem der Aktivität des interessierenden Polypeptids erreicht werden.

Analog dazu kann die Fermentation auch so gestaltet werden, dass unerwünschte Stoffwechselprodukte herausgefiltert oder durch Zugabe von Puffer oder jeweils passende Gegenionen neutralisiert werden.

Das hergestellte Polypeptid kann aus dem Fermentationsmedium geerntet werden. Ein solches Fermentationsverfahren ist gegenüber einer Isolation des Polypeptids aus der Wirtszelle, d. h. einer Produktaufbereitung aus der Zellmasse (Trockenmasse) bevorzugt, erfordert jedoch die Zurverfügungstellung von einem oder mehreren geeigneten Sekretionsmarkern bzw. -mechanismen und/oder Transportsystemen. Ohne Sekretion kann die Isolation des Polypeptids aus der Wirtszelle, d. h. eine Aufreinigung des Polypeptids aus der Zellmasse erfolgen. Auch hierfür sind verschiedene Verfahren bekannt, wie Fällung z. B. durch Ammoniumsulfat oder Ethanol, oder die chromatographische Reinigung.

Alle vorstehend ausgeführten Sachverhalte können zu Verfahren kombiniert werden, um erfindungsgemäß Polypeptide herzustellen. Es sind diesbezüglich eine Vielzahl von Kombinationsmöglichkeiten von Verfahrensschritten denkbar. Das optimale Verfahren muss für jeden konkreten Einzelfall ermittelt werden. Ferner kann bei den genannten Verfahren die in der Wirtszelle enthaltene erfindungsgemäße Nukleinsäure, insbesondere die Nukleinsäuresequenz (c), in einem, vorzugsweise mehreren Codons an die Codon-Usage der Wirtszelle angepasst worden sein.

Bevorzugt handelt es sich bei einem erfindungsgemäß hergestellten Polypeptid um ein Enzym. Besonders bevorzugt handelt es sich um ein Enzym, das ausgewählt ist aus der Gruppe bestehend aus Protease, Amylase, Cellulase, Hemicellulase, Mannanase, Tannase, Xylanase, Xanthanase, β-Glucosidase, Carrageenase, Oxidase, Oxidoreduktase, Lipase, oder Kombinationen hiervon. Ganz besonders bevorzugt handelt es sich bei dem Enzym um eines der vorstehend beschriebenen Enzyme.

Erfindungsgemäße Wirtszellen werden vorteilhaft in den beschriebenen erfindungsgemäßen Verfahren verwendet, um ein Polypeptid herzustellen. Konsequenterweise ist demnach ein weiterer Gegenstand der Erfindung die Verwendung einer vorstehend beschriebenen Wirtszelle zur Herstellung eines Polypeptids. Da erfindungsgemäße Wirtszellen einen erfindungsgemäßen Vektor bzw. eine erfindungsgemäße Nukleinsäure umfassen, sind weitere Gegenstände der Erfindung die Verwendung eines vorstehend beschriebenen Vektors zur Herstellung eines Polypeptids bzw. die Verwendung einer vorstehend beschriebenen Nukleinsäure zur Herstellung eines Polypeptids.

Alle Sachverhalte, Gegenstände und Ausführungsformen, die vorstehend beschrieben sind, sind auch auf diese Erfindungsgegenstände anwendbar. Daher wird an dieser Stelle ausdrücklich auf die Offenbarung an entsprechender Stelle verwiesen mit dem Hinweis, dass diese Offenbarung auch für die erfindungsgemäßen Verwendungen gilt (Verwendung der Wirtszelle bzw. des Vektors bzw. der Nukleinsäure).

Die nachfolgenden Beispiele erläutern die vorliegende Erfindung weiter, ohne sie darauf einzuschränken.

### Beispiele

Alle molekularbiologischen Arbeitsschritte folgen Standardmethoden, wie sie beispielsweise in dem Handbuch von Fritsch, Sambrook und Maniatis "Molecular cloning: a laboratory manual", Cold Spring Harbour Laborstory Press, New York, 1989, oder vergleichbaren einschlägigen Werken angegeben sind. Enzyme und Baukästen (Kits) wurden nach den Angaben der jeweiligen Hersteller eingesetzt.

Beispiel 1: Fermentative Produktion einer ersten Protease (leistungsverbesserte Variante der alkalischen Protease aus Bacillus lentus, die offenbart ist in EP 0701605, nachfolgend bezeichnet als Protease 1) mithilfe des Tandempromotors *P_{rpsB}-P_{subC}*

### a) Konstruktion des Produktionsplasmids

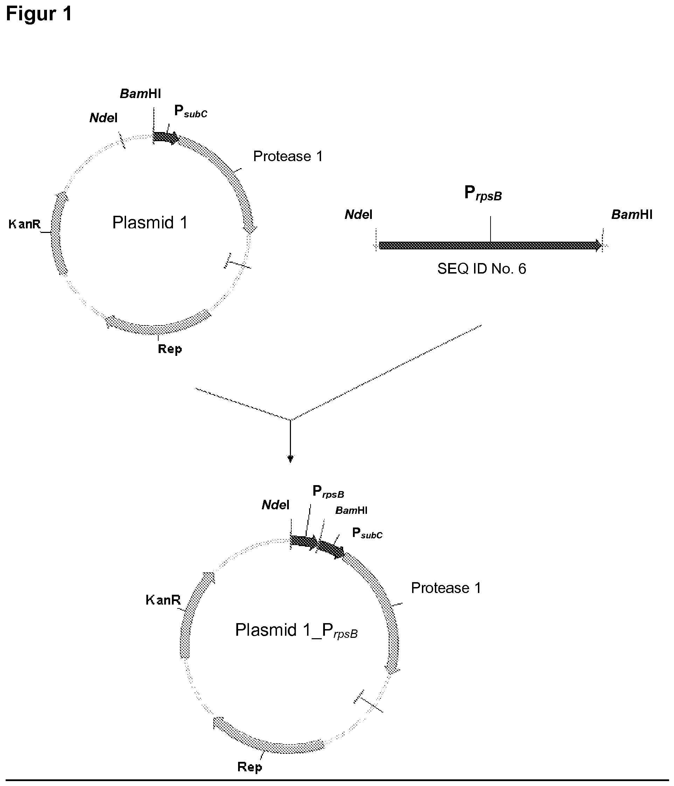

Ein 240 bp DNA-Fragment wurde synthetisiert (SEQ ID NO. 6), das den Promotor des Bacillus subtilis 168 rpsB-Gens umfasst (Positionen 7 bis 234) und von den Restriktionsschnittstellen Ndel und BamHI flankiert wird. Die Ligation des mit den Restriktionsendonukleasen Ndel und BamHI geschnittenen DNA-Fragments in den mit den gleichen Restriktionsendonukleasen geschnittenen Plasmid 1 resultierte in dem Plasmid 1_P*_{rpsB}* wie in Figur 1 angegeben. In dieses Plasmid befindet sich der rpsB-Promotor P*_{rpsB}* unmittelbar vor dem subC-Promotor P*_{subC}*, so dass beide Promotoren die Expression der Protease 1 kontrollieren (vgl. 1). Als subC-Promotor verwendet wurde der als "ATCC 53926 alkaline protease gene promotor" bezeichnete Promotor gemäß WO 91/02792. Ein Bacillus licheniformis-Stamm wurde mit dem Plasmid 1_P*_{rpsB}* transformiert, um den Protease 1 Produktionsstamm "Stamm 1" zu erhalten.

### b) Fermentative Produktion der Protease 1

Der Stamm 1 wurde in einem Standard Fermentationsverfahren eingesetzt. Verglichen mit dem Ausgangsstamm, der lediglich Plasmid 1 mit dem subC-Promotor ohne rpsB-Promotor beinhaltet, stieg die Ausbeute im 2 L-Laborfermenter um 24,1% (Mittelwert aus drei unabhängigen Fermentationen).

Beispiel 2: Fermentative Produktion einer zweiten Protease (Protease aus Bacillus pumilus gemäß WO 2007/131656, nachfolgend bezeichnet als Protease 2) mithilfe des Tandempromotors P*_{rpsB}*-P*_{subC}*

### a) Konstruktion des Produktionsplasmids

Das durch Restriktion mit den Restriktionsendonukleasen Clal und Sacl erhaltene ca. 1,3 kb DNA-Fragment des Protease 2-Produktionsplasmids Plasmid 2, welches die DNA-Sequenz des Propeptids und der reifen (mature) Protease 2 enthält, wurde in die entsprechenden Restriktionsschnittstellen des ca. 4,0 kb DNA-Fragments des Plasmids Plasmid 1_P*_{rpsB}* ligiert wie in 2 angegeben. Im resultierenden Plasmid 2_P*_{rpsB}* befindet sich der rpsB-Promotor R*_{rpsB}* unmittelbar vor dem subC-Promotor P*_{subC}*, so dass beide Promotoren nun die Expression der Protease 2 kontrollieren (vgl. 2). Als subC-Promotor verwendet wurde der als "ATCC 53926 alkaline protease gene promotor" bezeichnete Promotor gemäß WO 91/02792. Ein Bacillus licheniformis-Stamm wurde mit dem Plasmid 2_P*_{rpsB}* transformiert, um den Protease 2-Produktionsstamm "Stamm 2" zu erhalten.

### b) Fermentative Produktion der Protease 2

Der Stamm 2 wurde in einem Standard Fermentationsverfahren eingesetzt. Verglichen mit dem Ausgangsstamm, der lediglich Plasmid 2 mit dem subC-Promotor ohne rpsB-Promotor beinhaltet, stieg die Ausbeute im 2 L-Laborfermenter um 38% (Mittelwert aus 2 unabhängigen Fermentationen).

### Beschreibung der Figuren

- Figur 1:: Konstruktion des Plasmid 1_P*_{rpsB}* durch Insertion eines Ndel-BamHI-DNA-Fragments, das den rpsB-Promotor umfasst, in die entsprechenden Restriktionsschnittstellen des Protease 1-Produktionsplasmids "Plasmid 1".

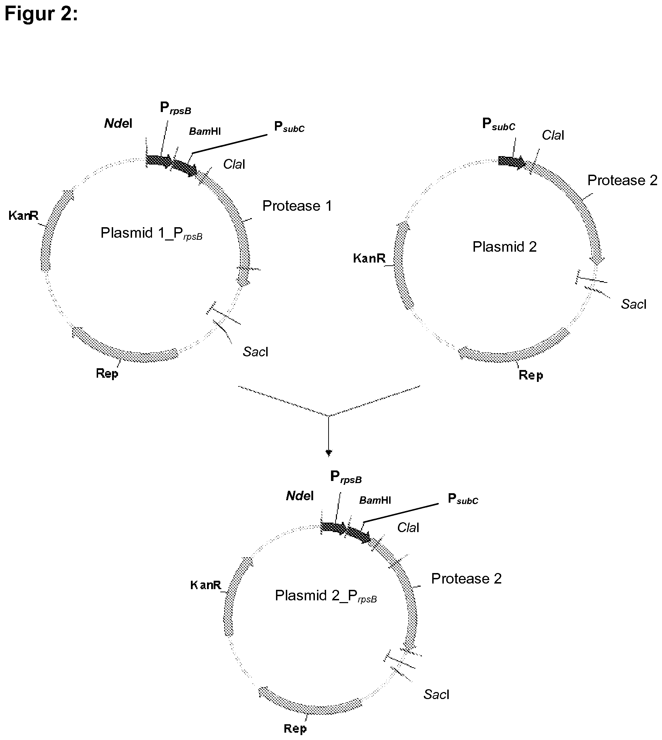

- Figur 2:: Konstruktion des Plasmid 2_P*_{rpsB}* durch Ligation des ca. 1,3 kb Clal-Sacl-DNA-Fragments, das die Gensequenz der Protease 2 umfasst, in die entsprechenden Restriktionsschnittstellen des Produktionsplasmids Plasmid 1_P*_{rpsB}* (ca. 4,0 kb DNA-Fragment).

## Patentansprüche

1. Nicht natürliche Nukleinsäure umfassend
a) eine erste Promotorsequenz umfassend eine Nukleinsäuresequenz, die zu SEQ ID NO. 7 zu mindestens 75% identisch ist,
b) eine zweite Promotorsequenz umfassend eine Nukleinsäuresequenz, die zu SEQ ID NO. 6 in den Positionen 7 bis 234 zu mindestens 78,5% identisch ist,
c) eine Nukleinsäuresequenz, die für ein Polypeptid codiert,
**dadurch gekennzeichnet, dass** sich auf dem Nukleinsäuremolekül in 5'→3'-Orientierung die zweite Promotorsequenz vor der ersten Promotorsequenz befindet.

2. Nukleinsäure nach Anspruch 1, **dadurch gekennzeichnet, dass** die erste Promotorsequenz und die zweite Promotorsequenz derart funktionell gekoppelt sind, dass sie eine verstärkte Expression der Nukleinsäuresequenz c) in einer Wirtszelle bewirken im Vergleich mit einer Wirtszelle, in der die Expression der Nukleinsäuresequenz c) mit nur einer der Promotorsequenzen a) oder b) erfolgt.

3. Nukleinsäure nach Anspruch 1 oder 2, **dadurch gekennzeichnet, dass** die erste Promotorsequenz natürlicherweise in Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans oder Bacillus pumilus vorhanden ist und/oder die zweite Promotorsequenz natürlicherweise in Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans oder Bacillus pumilus vorhanden ist.

4. Nukleinsäure nach einem der Ansprüche 1 bis 3, **dadurch gekennzeichnet, dass** die Nukleinsäuresequenz c) für ein Enzym codiert, insbesondere für eine Protease, Amylase, Cellulase, Hemicellulase, Mannanase, Tannase, Xylanase, Xanthanase, ß-Glucosidase, Carrageenase, Oxidase, Oxidoreduktase oder Lipase.

5. Vektor enthaltend eine Nukleinsäure nach einem der Ansprüche 1 bis 4, insbesondere ein Klonierungsvektor oder ein Expressionsvektor.

6. Nicht menschliche Wirtszelle enthaltend eine Nukleinsäure gemäß einem der Ansprüche 1 bis 4 oder einen Vektor gemäß Anspruch 5.

7. Wirtszelle nach Anspruch 6, **dadurch gekennzeichnet, dass** sie ein Bakterium ist, bevorzugt eines, das ausgewählt ist aus der Gruppe der Gattungen von Escherichia, Klebsiella, Bacillus, Staphylococcus, Corynebakterium, Arthrobacter, Streptomyces, Stenotrophomonas und Pseudomonas, weiter bevorzugt eines, das ausgewählt ist aus der Gruppe von Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor und Stenotrophomonas maltophilia.

8. Wirtszelle nach Anspruch 6, **dadurch gekennzeichnet, dass** sie einen Zellkern besitzt.

9. Verfahren zur Herstellung eines Polypeptids umfassend
a) Kultivieren einer Wirtszelle gemäß einem der Ansprüche 6 bis 8,
b) Isolieren des Polypeptids aus dem Kulturmedium oder aus der Wirtszelle.

10. Verwendung einer Wirtszelle gemäß einem der Ansprüche 6 bis 8 zur Herstellung eines Polypeptids.

11. Verwendung eines Vektors gemäß Anspruch 5 zur Herstellung eines Polypeptids.

12. Verwendung einer Nukleinsäure nach einem der Ansprüche 1 bis 4 zur Herstellung eines Polypeptids.

## Claims

1. A non-natural nucleic acid, comprising
a) a first promoter sequence comprising a nucleic acid sequence which has at least 75% identity to SEQ ID NO. 7,
b) a second promoter sequence comprising a nucleic acid sequence which has at least 78.5% identity to SEQ ID NO. 6 in positions 7 to 234,
c) a nucleic acid sequence which codes for a polypeptide,
wherein, in 5' → 3' orientation, the second promoter sequence is located upstream of the first promoter sequence on the nucleic acid molecule.

2. The nucleic acid according to claim 1, wherein the first promoter sequence and the second promoter sequence are functionally linked such that they bring about an expression of the nucleic acid sequence c) in a host cell which is enhanced in comparison with a host cell in which the expression of the nucleic acid sequence c) takes place with only one of the promoter sequences a) or b).

3. The nucleic acid according to claim 1 or 2, where-in the first promoter sequence is naturally present in Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans or Bacillus pumilus and/or the second promoter sequence is naturally present in Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans or Bacillus pumilus.

4. The nucleic acid according to any of claims 1 to 3, wherein the nucleic acid sequence c) codes for an enzyme, in particular for a protease, amylase, cellulase, hemicellulase, mannanase, tannase, xylanase, xanthanase, β-glucosidase, carrageenase, oxidase, oxidoreductase or lipase.

5. A vector comprising a nucleic acid according to any of claims 1 to 4, in particular a cloning vector or an expression vector.

6. A nonhuman host cell comprising a nucleic acid according to any of claims 1 to 4 or a vector according to claim 5.

7. The host cell according to claim 6, wherein the host cell is a bacterium, preferably one which is selected from the group of the genera of Eschericia, Klebsiella, Bacillus, Staphylococcus, Corynebacterium, Arthrobacter, Streptomyces, Stenotrophomonas and Pseudomonas, furthermore preferably one which is selected from the group of Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glatamicum, Arthrobacter oxidans,
Streptomyces lividans, Streptomyces coelicolor and Stenotrophomonas maltophilia.

8. The host cell according to claim 6, wherein the host cell has a nucleus.

9. A process for the preparation of a polypeptide comprising
a) culturing a host cell according to any of claims 6 to 8,
b) isolating the polypeptide from the culture medium or from the host cell.

10. The use of a host cell according to any of claims 6 to 8 for the preparation of a polypeptide.

11. The use of a vector according to claim 5 for the preparation of a polypeptide.

12. The use of a nucleic acid according to any of claims 1 to 4 for the preparation of a poly-peptide.

## Revendications

1. Acide nucléique non-naturel comprenant
a) une première séquence promotrice, comprenant une séquence d'acide nucléique ayant une identité d'au moins 75 % avec SEQ ID NO:7,
b) une deuxième séquence promotrice, comprenant une séquence d'acide nucléique ayant une identité d'au moins 78,5 % avec SEQ ID NO:6 sur les positions 7 à 234,
c) une séquence d'acide nucléique qui code pour un polypeptide,
**caractérisé en ce que** la deuxième séquence promotrice se trouve, sur la molécule d'acide nucléique, avant la première séquence promotrice selon l'orientation 5'→3'.

2. Acide nucléique selon la revendication 1, **caractérisé en ce que** la première séquence promotrice et la deuxième séquence promotrice sont fonctionnellement couplées de façon à occasionner une expression de la séquence d'acide nucléique c) dans une cellule hôte, qui est renforcée par comparaison avec une cellule hôte dans laquelle l'expression de la séquence d'acide nucléique c) a lieu avec uniquement l'une des séquences promotrices a) ou b).

3. Acide nucléique selon la revendication 1 ou 2, **caractérisé en ce que** la première séquence promotrice est naturellement présente dans Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans ou Bacillus pumilus, et/ou que la deuxième séquence promotrice est naturellement présente dans Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans ou Bacillus pumilus.

4. Acide nucléique selon l'une des revendications 1 à 3, **caractérisé en ce que** la séquence d'acide nucléique c) code pour une enzyme, en particulier pour une protéase, une amylase, une cellulase, une hémicellulase, une mannanase, une tannase, une xylanase, une xanthanase, une β-glucosidase, une carragénase, une oxydase, une oxydoréductase ou une lipase.

5. Vecteur contenant un acide nucléique selon l'une des revendications 1 à 4, en particulier un vecteur de clonage ou un vecteur d'expression.

6. Cellule hôte non-humaine contenant un acide nucléique selon l'une des revendications 1 à 4 ou un vecteur selon la revendication 5.

7. Cellule hôte selon la revendication 6, **caractérisée en ce qu'**il s'agit d'une bactérie, de préférence d'une bactérie qui est choisie dans le groupe des genres Escherichia, Klebsiella, Bacillus, Staphylococcus, Corynebacterium, Arthrobacter, Streptomyces, Stenotrophomonas et Pseudomonas, d'une manière plus préférée d'une bactérie qui est choisie dans le groupe consistant en Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus canosus, Corynebacterium glutamicum, Arthrobacter oxydans, Streptomyces lividans, Streptomyces coelicolor et Stenotrophomonas maltophilia.

8. Cellule hôte selon la revendication 6, **caractérisée en ce qu'**elle possède un noyau cellulaire.

9. Procédé de fabrication d'un polypeptide, comprenant
a) la culture d'une cellule hôte selon l'une des revendications 6 à 8,
b) l'isolement du polypeptide à partir du milieu de culture ou de la cellule hôte.

10. Utilisation d'une cellule hôte selon l'une des revendications 6 à 8 pour la fabrication d'un polypeptide.

11. Utilisation d'un vecteur selon la revendication 5 pour la fabrication d'un polypeptide.

12. Utilisation d'un acide nucléique selon l'une des revendications 1 à 4 pour la fabrication d'un polypeptide.


### SEQUENCE LISTING

<110> Henkel AG & Co. KGaA
<120> Expressionsverstärkte Nukleinsäuren
<130> H 08172
<160> 7
<170> PatentIn version 3.3
<210> 1
   <211> 6
   <212> DNA
   <213> Artificial
<220>
   <223> -35-Region Konsensus
<400> 1
   ttgaca 6
<210> 2
   <211> 6
   <212> DNA
   <213> Artificial
<220>
   <223> -10-Region Konsensus
<400> 2
   tataat 6
<210> 3
   <211> 6
   <212> DNA
   <213> Artificial
<220>
   <223> -10-Region subC
<400> 3
   tacaat 6
<210> 4
   <211> 6
   <212> DNA
   <213> Artificial
<220>
   <223> -35-Region subC
<400> 4
   tactaa 6
<210> 5
   <211> 274
   <212> PRT
   <213> Bacillus licheniformis
<400> 5
   Ala Gln Thr Val Pro Tyr Gly Ile Pro Leu Ile Lys Ala Asp Lys Val
   1               5                   10                  15
   Gln Ala Gln Gly Phe Lys Gly Ala Asn Val Lys Val Ala Val Leu Asp
               20                  25                  30
   Thr Gly Ile Gln Ala Ser His Pro Asp Leu Asn Val Val Gly Gly Ala
           35                  40                  45
   Ser Phe Val Ala Gly Glu Ala Tyr Asn Thr Asp Gly Asn Gly His Gly
       50                  55                  60
   Thr His Val Ala Gly Thr Val Ala Ala Leu Asp Asn Thr Thr Gly Val
   65                  70                  75                  80
   Leu Gly Val Ala Pro Ser Val Ser Leu Tyr Ala Val Lys Val Leu Asn
                   85                  90                  95
   Ser Ser Gly Ser Gly Thr Tyr Ser Gly Ile Val Ser Gly Ile Glu Trp
               100                 105                 110
   Ala Thr Thr Asn Gly Met Asp Val Ile Asn Met Ser Leu Gly Gly Pro
           115                 120                 125
   Ser Gly Ser Thr Ala Met Lys Gln Ala Val Asp Asn Ala Tyr Ala Arg
       130                 135                 140
   Gly Val Val Val Val Ala Ala Ala Gly Asn Ser Gly Ser Ser Gly Asn
   145                 150                 155                 160
   Thr Asn Thr Ile Gly Tyr Pro Ala Lys Tyr Asp Ser Val Ile Ala Val
                   165                 170                 175
   Gly Ala Val Asp Ser Asn Ser Asn Arg Ala Ser Phe Ser Ser Val Gly
               180                 185                 190
   Ala Glu Leu Glu Val Met Ala Pro Gly Ala Gly Val Tyr Ser Thr Tyr
           195                 200                 205
   Pro Thr Ser Thr Tyr Ala Thr Leu Asn Gly Thr Ser Met Ala Ser Pro
       210                 215                 220
   His Val Ala Gly Ala Ala Ala Leu Ile Leu Ser Lys His Pro Asn Leu
   225                 230                 235                 240
   Ser Ala Ser Gln Val Arg Asn Arg Leu Ser Ser Thr Ala Thr Tyr Leu
                   245                 250                 255
   Gly Ser Ser Phe Tyr Tyr Gly Lys Gly Leu Ile Asn Val Glu Ala Ala
               260                 265                 270
   Ala Gln
<210> 6
   <211> 240
   <212> DNA
   <213> Artificial
<220>
   <223> PrpsB
<400> 6
   catatgccag gtcatagaat tatatgaaca gggatattcg gcaagtcaaa ttgcccagaa     60
   aatgaagagc ggaaagacag aaatcgagct atttttaaaa tttcgctcca aaggtgtaaa    120
   ggattcttga ttgtcggata aagctgtgtt atattatgtc ttggtgttaa atacacacgc    180
   ttaacgattt atgcagaggg tgctgcaggc ggcagttctg tacaaaaatg acctggatcc    240
<210> 7
   <211> 156
   <212> DNA
   <213> Artificial
<220>
   <223> Sequenzabschnitt gemäß Figur 27 der WO91/02792
<400> 7
   ctcgggacct ctttccctcg gaggctgaag cggtctattc atactttcga actgaacatt     60
   tttctaaaac agttattaat aaccaaaaaa ttttaaattg gtcctccaaa aaaataggcc    120
   taccatataa ttcatttttt ttctataata aattaa                              156